# EUROPEAN PATENT APPLICATION

(11) **EP 4 643 928 A1**
(43) Date of publication of application: **05.11.2025**
(21) Application number: 25174046.0
(22) Date of filing: 02.05.2025
(51) Int. Cl.: A61N 1/04, A61B 5/00, A61N 1/36

(54) **WEARABLE HEADBAND DEVICE FOR NON-INVASIVE BRAIN STIMULATION**

(30) Priority: 03.05.2024 IN 202411035193
(71) Applicant: Neuracle Health Private Limited, New Delhi 110074 (IN)
(72) Inventor: Sahni, Lakshay, 110074 New Delhi (IN); Kanwar, Swapniil, 110074 New Delhi (IN); Yellparagada, Ramya, 110074 New Delhi (IN)
(74) Representative: Nirwan, Prajwal

(57) **Abstract**

A wearable headband device for non-invasive brain stimulation includes a first stimulation electrode **(102A),** a second stimulation electrode **(102B)** and an electronic circuit **(106)** for switching a direction of flow of current between the first stimulation electrode **(102A)** and the second stimulation electrode **(102B).** The electronic circuit **(106)** includes a first circuit **(116)** including a pair of first type of switches **(118)** and a pair of second type of switches **(120).** Furthermore, a protection circuit **(124)** is configured to detect and control the flow of current passing through the first stimulation electrode **(102A)** and the second stimulation electrode **(102B).** Further, a microcontroller **(126)** is configured to control the pair of first type of switches **(118)** and the pair of second type of switches **(120)** of the first circuit **(116)** for the switching the direction of flow of current between the first stimulation electrode **(102A)** and the second stimulation electrode **(102B).**

## Description

### TECHNICAL FIELD

The present disclosure relates generally to the field of non-invasive brain stimulation and brain imaging and more specifically, to a wearable headband device for non-invasive brain stimulation and brain imaging with an advanced electronic circuit for significantly improved user experience, multiple electrical stimulation waveforms and safety of users wearing the wearable headband device.

### BACKGROUND

The brain is made up of billions of interconnected neurons and other cell types in connected networks. This complex network of neurons is responsible for processing sensory input, generating motor commands, and controlling various behaviours and cognitive functions. Stimulation technologies that affect electric fields and electrochemical signalling in neurons can modulate the pattern of neural activity and cause altered behaviour, cognitive states, perception, and motor output. Existing brain stimulation devices and systems are mostly designed, developed, and mostly focussed on a hospital use case with large systems setups, which requires operation by a trained specialist. Further, the conventional stimulation devices and systems are very bulky in size and are mostly non-portable units that are difficult to carry due to which such systems are costly and employed only in a limited number of hospitals. Furthermore, with some recent advances some portable devices have been developed, but still are bulky and mostly cover the entire scalp of the brain in the form of a cap like wearable units. However, such systems still require a connection to large analysers to carry out any meaningful analysis of a health state and for non-invasive brain stimulation.

Traditionally, brain stimulation systems include electrical or sensory stimulation, such as electroconvulsive therapy (ECT)are big in size, bulky, complex, costly, which is not desirable. Furthermore, traditional brain stimulation systems that perform testing and determining stimulation points are time-consuming that are found to affect patient cooperation. With conventional wearable devices that are involved in handling of current for stimulation purpose there is further a valid concern of user safety. Therefore, there exists a technical problem of how to develop an advanced and easy-to-use wearable device with advanced electronics that can solve the problem of overcurrent flow and user safety.

Therefore, in light of the foregoing discussion, there exists a need to overcome the aforementioned drawbacks associated with the conventional methods and systems of non-invasive brain stimulation.

### SUMMARY

The present disclosure provides a wearable headband device for non-invasive brain stimulation. The present disclosure provides a solution to the existing problem of how to detect and control the overcurrent flow and other potential safety hazards in the electronic devices that are used for the traditional stimulation methods for brain stimulation. An objective of the present disclosure is to provide a solution that overcomes at least partially the problems encountered in the prior art and provides an improved wearable headband device for non-invasive brain stimulation with minimum components and reduced cost.

One or more objectives of the present disclosure are achieved by the solutions provided in the enclosed independent claims. Advantageous implementations of the present disclosure are further defined in the dependent claims.

In one aspect, the present disclosure provides a wearable headband device for non-invasive brain stimulation, comprising a first stimulation electrode and a second stimulation electrode and an electronic circuit for switching the direction of flow of current between the first stimulation electrode and the second stimulation electrode and also control the amount of current between the first stimulation electrode and second stimulation electrode. The electronic circuit comprises a first circuit that comprises a pair of first type of switches and a pair of second type of switches that are electrically connected to each other, wherein the first circuit is further electrically connected to the first stimulation electrode and the second stimulation electrode. The electronic circuit further comprises a plurality of second circuits configured to control an operation of the pair of first type of switches and the pair of second type of switches of the first circuit. The electronic circuit further comprises a protection circuit that is configured to detect and control the flow of current passing through the first stimulation electrode and the second stimulation electrode via the first circuit, based on a voltage drop across a current sense circuit of the protection circuit. The electronic circuit further comprises a microcontroller that is configured to control the pair of first type of switches and the pair of second type of switches of the first circuit through the plurality of second circuits for the switching the direction of flow of current between the first stimulation electrode and the second stimulation electrode in the non-invasive brain stimulation it also gives instruction for amount of current to be flown between the first stimulation electrode and the second stimulation electrode.

The disclosed wearable headband device is a non-invasive brain stimulation device, which means the wearable headband device does not require any surgical procedures for non-invasive brain stimulation. Beneficially, the development of a new circuit (referred to as a first circuit) that comprises the low-power switches enables to switch the direction of current flow between the first stimulation electrode and the second stimulation electrode. This in turn allowing for a more targeted and effective stimulation with improved accuracy. Further, the plurality of second circuits is designed and developed to accurately control an operation of the pair of first type of switches and the pair of second type of switches of the first circuit, for example, to turn ON or OFF a first type of switch and a second type of switch at the same time (or within a few microseconds) based on command from the microcontroller. This provides a capability to control the switching of the direction of current flow between the first stimulation electrode and the second stimulation electrode and raising errors if anything goes wrong. Furthermore, the wearable headband device includes the protection circuit that is used to prevent overcurrent flow and ensure patient safety at a reduced cost, thereby providing a technical advancement as well as providing the wearable headband device that is economically viable. Advantageously, the invention employs another new circuit specifically developed to ensure safety for users wearing the wearable headband device by automatically detecting and controlling the flow of current passing through the first stimulation electrode and the second stimulation electrode via the first circuit, based on the voltage drop across a current sense circuit of the protection circuit.

It has to be noted that all devices, elements, circuitry, units and means described in the present application could be implemented in the software or hardware elements or any kind of combination thereof. All steps which are performed by the various entities described in the present application as well as the functionalities described to be performed by the various entities are intended to mean that the respective entity is adapted to or configured to perform the respective steps and functionalities. Even if, in the following description of specific embodiments, a specific functionality or step to be performed by external entities is not reflected in the description of a specific detailed element of that entity which performs that specific step or functionality, it should be clear for a skilled person that these methods and functionalities can be implemented in respective software or hardware elements, or any kind of combination thereof. It will be appreciated that features of the present disclosure are susceptible to being combined in various combinations without departing from the scope of the present disclosure as defined by the appended claims.

Additional aspects, advantages, features, and objects of the present disclosure would be made apparent from the drawings and the detailed description of the illustrative implementations construed in conjunction with the appended claims that follow.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:
FIG. 1A is a block diagram of a wearable headband device for non-invasive brain stimulation, in accordance with an embodiment of the present disclosure;
FIG. 1B is a block diagram that depicts a first printed circuit board (PCB) of a wearable headband device for non-invasive brain stimulation, in accordance with an embodiment of the present disclosure;
FIG. 2 is a diagram that depicts various exemplary components of a brain stimulation circuit, in accordance with an embodiment of the present disclosure; and
   FIGs. 3A and 3B are different diagrams that depict different exemplary scenarios of a wearable headband device for non-invasive brain stimulation to be worn by a user, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

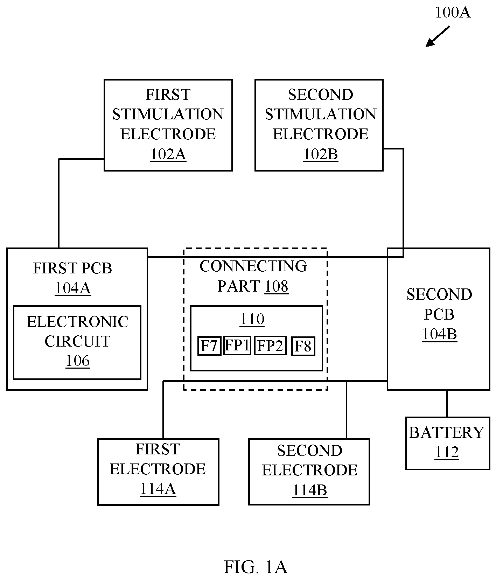

FIG. 1A is a block diagram of a wearable headband device for non-invasive brain stimulation, in accordance with an embodiment of the present disclosure. With reference to the FIG. 1A, there is shown a block diagram of a wearable headband device **100A** for non-invasive brain stimulation. The wearable headband device **100A** includes a first stimulation electrode **102A** and a second stimulation electrode **102B.** There is further shown a first printed circuit board (PCB) **104A**, a second PCB **104B,** and an electronic circuit **106.**

The wearable headband device **100A** is used for non-invasive brain stimulation, which uses low-intensity electrical currents to stimulate specific regions of the brain. The wearable headband device **100A** is typically designed to be worn on the head of a user and can be used to treat a variety of conditions, including but not limited to depression, anxiety, migraine, mild cognitive impairment (MCI), and chronic pain.

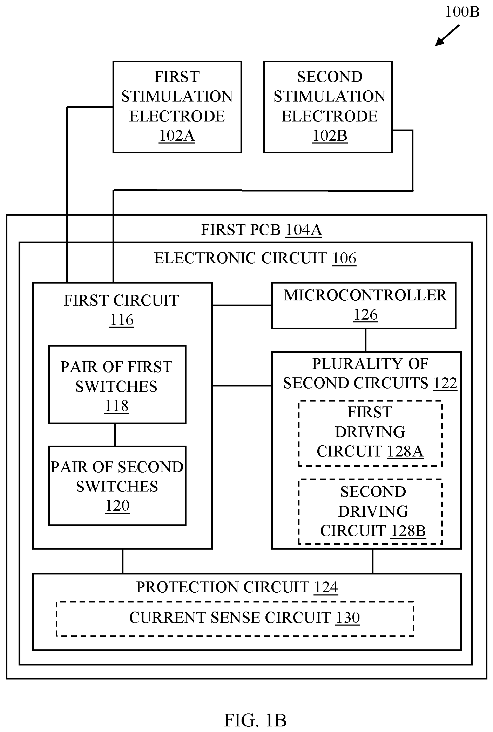

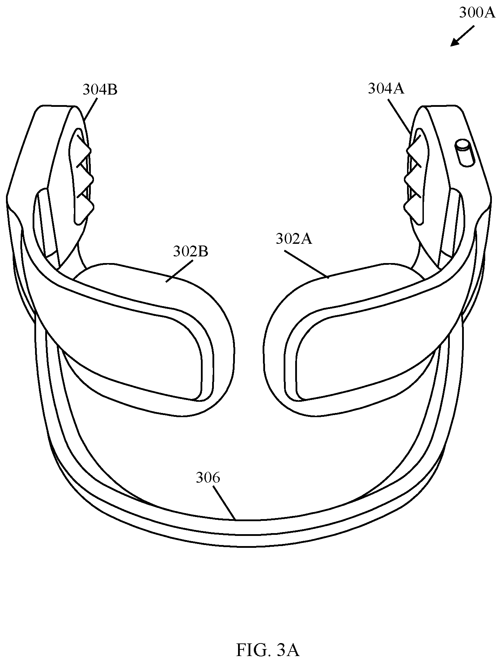

The wearable headband device **100A** includes two earpieces, such as a left earpiece and a right earpiece, as further shown and described in FIG. 3A. Moreover, each earpiece includes a separate PCB. For example, the right earpiece includes the first PCB **104A** and the left earpiece includes the second PCB **104B.** In another example, the right earpiece includes the second PCB **104B** and the left earpiece includes the first PCB **104A.** In addition, the first PCB **104A** is configured to control the first stimulation electrode **102A** and the second stimulation electrode **102B.** In an example, each of the first stimulation electrode **102A** and the second stimulation electrode **102B** includes a conductive electrode, a conductive glue, a metal electrode, and a joint. Moreover, the first stimulation electrode **102A** is directly connected to the first PCB **104A,** and the second stimulation electrode **102B** is connected to the first PCB **104A** through a connecting part **108.** For example, the second stimulation electrode **102B** is connected to the first PCB **104A** through an electric wire, which passes through the left earpiece and also through the connecting part **108.** There is further shown that the first PCB **104A** includes the electronic circuit **106,** which is configured for switching a direction of flow of current between the first stimulation electrode **102A** and the second stimulation electrode **102B,** as further shown and described in FIG. 1B. The electronic circuit **106** is used for precise control of current flow to the first stimulation electrode **102A** and the second stimulation electrode **102B** without causing fluctuations and protects overcurrent flow, ensuring the safety of a patient. In an example, each of the first stimulation electrode **102A** and the second stimulation electrode **102B** corresponds to a transcranial electrical stimulation (tES) electrode. Moreover, each of the first stimulation electrode **102A,** and the second stimulation electrode **102B,** uses an electrical current to modulate the activity of specific regions of the brain.

In an implementation, the second PCB **104B** includes another circuit that is used for electroencephalography (EEG), which is a non-invasive technique used to measure the electrical activity of the brain via electrodes placed on the scalp, such as through EEG electrode circuit **110** that are arranged in the connecting part **108.** There are further shown four numbers of frontal lobes (i.e., F7, FP1, FP2, and F8) that indicate specific locations on a scalp where the EEG electrode circuit **110** is placed. Such specific locations are used for EEG electrode placement to ensure consistent and comparable results. However, the points or the specific locations on the scalp where the EEG electrode circuit **110** is placed may change without affecting the scope of the present disclosure. Moreover, the second PCB **104B** is further configured to control a first electrode **114A** and a second electrode **114B.** In an example, the first electrode **114A** is a reference electrode (i.e., SRB1 electrode) and the second electrode **114B** a BIAS electrode, which is used in EEG studies. In an implementation, the second PCB **104B** is configured to receive current from a battery **112** and transfer the current to the first PCB **104A** through the connecting part **108** (e.g., using electric wires). Moreover, the second PCB **104B** is also configured to use the battery **112** to supply current to EEG electrode circuit **110,** the first electrode **114A,** and the second electrode **114B.** In an example, the value of the battery **112** ranges from 2 volts (V) to 5 volts. In another example, the value of the battery **112** ranges from 2.8 V to 4.2 V.

The wearable headband device **100A** for non-invasive brain stimulation also prevents the overflow of current when providing stimulation. The wearable headband device **100A** includes the first stimulation electrode **102A,** the second stimulation electrode **102B,** and the electronic circuit **106** for switching the direction of flow of current between the first stimulation electrode **102A** and the second stimulation electrode **102B.** The electronic circuit **106** is configured to track the flow of current with respect to an ideal wave, control current parameters as per Bluetooth command, and notify the user about any error safely. In addition, the electronic circuit **106** is also configured to stop the flow of current during any short circuit or an open circuit. The electronic circuit **106** is used for precise control of current flow to the electrodes without causing fluctuations. Additionally, the electronic circuit **106** is used for accurate control of current flow to different electrodes and protects overcurrent flow, ensuring the safety of the patient.

FIG. 1B is a block diagram that depicts a first printed circuit board (PCB) of a wearable headband device for non-invasive brain stimulation, in accordance with an embodiment of the present disclosure. FIG. 1B is described in conjunction with elements from FIG. 1A. With reference to FIG. 1B, there is shown a block diagram **100B** that depicts the first PCB **104A** of the wearable headband device **100A** for non-invasive brain stimulation. The first PCB **104A** includes the electronic circuit **106.**

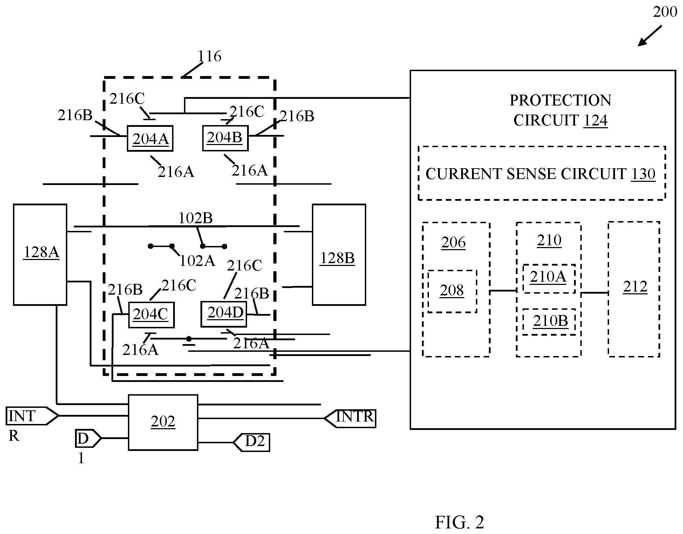

In operation, the electronic circuit **106** is configured for switching a direction of the flow of current between the first stimulation electrode **1020A** and the second stimulation electrode **102B.** The electronic circuit **106** includes a first circuit **116** that includes a pair of first type of switches **118** and a pair of second type of switches **120** that are electrically connected to each other. Moreover, the first circuit **116** is further electrically connected to the first stimulation electrode **102A** and the second stimulation electrode **102B,** as shown in FIG. 1B. In an implementation, the first circuit **116** is a modified H-bridge circuit that includes the pair of first type of switches **118** and the pair of second type of switches **120,** as further shown and described in FIG. 2. In an implementation, the first circuit **116** is further connected with the battery **112** that acts as a power source and is connected to the second PCB **104B** (of FIG. 1A). In such implementation, the first circuit **116** is preferably connected with the battery **112** through a voltage converter. In an example, a voltage of the battery **112** ranges from 2 volts to 5 volts. In another example, the voltage of the battery **112** preferably ranges from 2.8 volts to 4.2 volts. However, the voltage of the battery **112** can vary without limiting the scope of the present disclosure. Moreover, a voltage converter is used to receive voltage from the battery **112** and provide an improved voltage value (e.g., up to 20 volts) to the first PCB **104A** and a voltage value of 3v to the first PCB **104A** and to the second PCB **104B.** In addition, the first circuit **116** is configured to steer the flow of current from the power source toward the first stimulation electrode **102A** and the second stimulation electrode **102B** via the pair of first type of switches **118** and the pair of second type of switches **120** when the wearable headband device **100A** receives a command from a user device (e.g., from a mobile phone) through the microcontroller **126.** Therefore, if the wearable headband device **100A** receives the command from the user (e.g., through a user device), then the modified H-bridge circuit is configured to steer the flow of current toward the first stimulation electrode **102A** and the second stimulation electrode **102B** via the pair of first type of switches **118** and the pair of second type of switches **120.**

The electronic circuit **106** further includes a plurality of second circuits **122** that are configured to control an operation of the pair of first type of switches **118** and the pair of second type of switches **120** of the first circuit **116.** However, the electronic circuit **106** can also be used in a non-wearable devices or invasive brain simulations without affecting the scope of the present disclosure. In an example, the plurality of second circuits **122** corresponds to H-bridge driver circuits that are configured to control (or drive) the operation of the first circuit **116,** such as to control the flow of current through the first stimulation electrode **102A** and the second stimulation electrode **102B.** The electronic circuit 106 further includes a protection circuit **124** that is configured to detect and control the flow of current passing through the first stimulation electrode **102A** and the second stimulation electrode **102B** via the first circuit **116,** based on a voltage drop across the current sense circuit **130** in the protection circuit **124.** The protection circuit **124** is beneficial to ensure that the current flow passing through the first stimulation electrode **102A** and the second stimulation electrode **102B** remains within safe limits and also ensures the safety of a user by preventing any potential harm from excessive current flow.

The electronic circuit **106** further includes a microcontroller **126** that is configured to control the pair of first type of switches **118** and the pair of second type of switches **120** of the first circuit **116** through the plurality of second circuits **122** for the switching the direction of flow of current between the first stimulation electrode **102A** and the second stimulation electrode **102B** in the non-invasive brain stimulation. Therefore, the microcontroller **126** is used for precise and controlled switching of the current flow direction between the first stimulation electrode **102A** and the second stimulation electrode **102B** in the non-invasive brain stimulation. In an implementation, the switching of the direction of the flow of current further includes reversing a polarity or the direction of the flow of current between the first stimulation electrode **102A** and the second stimulation electrode **102B.** As a result, the electronic circuit **106** allows for improved and specific targeting of the brain stimulation specific areas of the brain, as well as the electronic circuit **106** can vary the stimulation intensity and duration. In addition, the microcontroller **126** can also be programmed to perform specific stimulation protocols, such as alternating between different current flow directions at specific intervals or in response to external triggers, such as a patient's brain activity. Additionally, the microcontroller **126** can communicate with an external device, such as a computer or smartphone, to receive input from the patient or to provide information on the stimulation parameters and effects. As a result, the use of the microcontroller **126** in the electronic circuit **106** provides a high level of precision and control in the non-invasive brain stimulation process. In an implementation, the microcontroller **126** is further configured to detect a fault in the electronic circuit **106** and open the pair of first type of switches **118** and the pair of second type of switches **120** of the first circuit **116** in response to the fault. In an example, the microcontroller **126** is programmed with a set of fault detection algorithms that continuously monitor the electronic circuit **106** for any potential faults. The set of fault detection algorithms are used by the microcontroller **126** to detect abnormal voltage, abnormal current levels, abnormal temperature variations, or other abnormal parameters that could indicate the fault in the electronic circuit **106.** Thereafter, the microcontroller **126** is configured to open the pair of first type of switches **118** and the pair of second type of switches **120** to interrupt the flow of current through the first stimulation electrode **102A** and the second stimulation electrode **102B,** effectively shutting down the electronic circuit **106** until the fault is resolved. In an example, the microcontroller **126** is further configured to shut down the first circuit **116,** the plurality of second circuits **122,** and the protection circuit **124** to protect the patient. As a result, the microcontroller **126** is beneficial to ensure the safety of the user by preventing any potential harm caused by a malfunctioning condition in the electronic circuit **106** and protecting the user from the overcurrent simulation.

In accordance with an embodiment, the protection circuit **124** is configured to cut off the overcurrent passing to the first stimulation electrode **102A** and the second stimulation electrode **102B** without any intervention of the microcontroller **126.** The overcurrent passed through the electrodes (i.e., the first simulation electrode **102A** and the second simulation electrode **102B)** could potentially harm the user. Therefore, by automatically cutting off the current without waiting for instructions from the microcontroller **126,** the wearable headband device **100A** can swiftly prevent any potential risks to the user's well-being.

In an implementation, the switching of the current flow is performed based on demographic data, scores on tests and/or a change in a pattern of electroencephalography (EEG) data and based on one or more transcranial electrical stimulation (tES) application criteria when the wearable headband device **100A** is worn by a user. In an example, the tES application criteria further may also include but are not limited to tDCS (transcranial direct current stimulation), tACS (transcranial alternating current stimulation), tRNS (transcranial random noise stimulation), tPCS (transcranial pulsating current stimulation), and the like. Moreover, the wearable headband device **100A** for non-invasive brain stimulation may also be referred to as an EEG-controlled transcranial Electrical stimulation (tES) headband. In an example, the wearable headband device **100A** includes EEG sensors that are used to measure the electrical activity of the brain, which can be used to detect changes in brain activity patterns. Moreover, the microcontroller **126** is configured to analyze the EEG data in real-time and detect changes in the brain activity patterns, such as based on the detected changes in the brain activity patterns, the microcontroller **126** can adjust the current flow or the direction through the stimulation electrodes. Optionally, the microcontroller **126** is configured to collect the EEG data in real-time and transmit the data to the cloud for processing through the paired computer or smartphone or on the paired computer/phone for detecting changes in the brain activity patterns. Moreover, the switching of the amount of the current flow can be manually set by an operator. As a result, the microcontroller **126** allows the wearable headband device **100A** to adapt the stimulation parameters in real-time, providing a more personalized and effective stimulation. In addition, the wearable headband device **100A** is also configured to switch the current flow based on the one or more tES application criteria, such as FALL-D parameters (e.g., frequency, amplitude, latency, location), the duration of stimulation (D), duty cycle, interpulse interval, the intensity of the current. The microcontroller **126** is programmed with the one or more tES application criteria and adjusts the current flow accordingly to ensure that the stimulation is delivered according to the one or more tES application criteria, which may be beneficial to allow a more personalized and effective non-invasive brain stimulation.

In accordance with an embodiment, the protection circuit **124** is further configured to provide overvoltage protection between the first stimulation electrode **102A** and the second stimulation electrode **102B.** The protection circuit **124** is configured to detect the flow of current passing through the first simulation electrode **102A** and the second simulation electrode **102B** based on the voltage drop across the current sense circuit **130.** If an overvoltage condition occurs, where the voltage drop exceeds a predefined threshold, the protection circuit **124** cuts off the overcurrent flowing to the first simulation electrode **102A** and the second simulation electrode **102B.** As a result, by automatically cutting off the voltage between the first simulation electrode **102A** and the second simulation electrode **102B,** the wearable headband device **100A** can swiftly prevent any potential risks to the user's well-being.

In an implementation, the microcontroller **126** is configured for the switching the direction of the flow of current between the first stimulation electrode **102A** and the second stimulation electrode **102B** in the non-invasive brain stimulation at a frequency of 10Hz for a session of a desired time period, such as for 20 minutes (i.e., frequency of switching is 10 times per second). However, the frequency may vary from one user to another user for different sessions. In an example, the frequency ranges from 8 Hz to 16 Hz for 20 minutes. In another example, the frequency ranges from 10 Hz to 20 Hz for 30 minutes, and the like. In an example, a waveform of the frequency for the non-invasive brain stimulation may be a pulsating waveform, a direct current waveform, a variable waveform, and an alternating waveform. Optionally, the pulsating waveform can be a pulsating sine waveform, a pulsating triangular waveform, a pulsating square waveform, and a pulsating complex waveform. Similarly, the alternating waveform can be an alternating sine waveform, an alternating triangular waveform, an alternating square waveform, and an alternating complex waveform. However, other combinations of the waveform for non-invasive brain stimulation can be selected without limiting the scope of the invention.

In an implementation, the plurality of second circuits **122** includes a first driving circuit **128A** that is configured to control a switch of the pair of second type of switches **120** and further control another switch of the pair of first type of switches **118** when a first command is received via a first pin of the microcontroller, as further shown and described in FIG. 2. In such implementation, the plurality of second circuits **122** further a second driving circuit **128B** that is configured to control yet another switch of the pair of second type of switches **120** and further control another switch of the pair of first type of switches **118** when a second command is received via a second pin of the microcontroller **126,** as further shown and described in FIG. 2. In other words, the first driving circuit **128A** and the second driving circuit **128B** are configured to control the pair of first type of switches **118** and the pair of second type of switches **120** when commands are received via the microcontroller **126,** such as by selectively turning on and off the pair of first type of and second type of switches. By controlling the switching of the pair of first type of switches **118** and the pair of second type of switches **120,** the plurality of second circuits **122** can control the direction of current flow through the first stimulation electrode **102A** and the second stimulation electrode **102B.** As a result, the wearable headband device **100A** allows for precise control of the current flow through the use of the plurality of second circuits **122** and the microcontroller **126.**

In an implementation, the flow of current that passes through the first stimulation electrode **102A** and the second stimulation electrode **102B** causes a potential difference across a current sense circuit **130** of the protection circuit **124.** In an example, the current sense circuit **130** is configured to determine an overcurrent passing through the first stimulation electrode **102A** and the second stimulation electrode **102B** based on the potential difference caused across the current sense circuit **130** of the protection circuit **124.** In such implementation, the protection circuit **124** is further configured to determine if a voltage drop exceeds a pre-defined threshold, and in response to an increased voltage drop, the protection circuit **124** is configured to cut off the overcurrent passing through the first stimulation electrode **102A** and the second stimulation electrode **102B.** In an example, the protection circuit **124** is configured to monitor the potential difference in real-time and compares the potential difference to a pre-defined threshold (i.e., a safe voltage limit) so as to determine if the voltage drop exceeds the pre-defined threshold or not. Moreover, if the voltage drop exceeds the safe limits, then the protection circuit **124** is configured to immediately interrupt the current flow and trigger an alarm to alert the user. As a result, the protection circuit **124** prevents any potential harm from excessive current flow and ensures the safety of the user. In such implementation, the protection circuit **124** is configured to cut off the overcurrent independent of receipt of any command from the microcontroller **126,** as further shown and described in FIG. 2. Beneficially as compared to conventional approaches, the protection circuit **124** is designed to ensure safety automatically and independently for the user. In such implementation, the protection circuit **124** is configured to cut off the overcurrent passing to the first stimulation electrode **102A** and the second stimulation electrode **102B** without depending on a polarity of the flow of current across the first stimulation electrode **102A** and the second stimulation electrode **102B.** As a result, the protection circuit **124** provides an added level of security for different users, for example, by safeguarding the patient from overcurrent, as the protection circuit **124** can detect and respond to potential hazards without the need for manual intervention. Furthermore, if the current bypassing circuit **212** in the protection circuit **124** fails to operate, then, in that case, a secondary check is performed by the AND gate **202.** Thereafter, the microcontroller **126** is configured to send a stop command to the AND gate **202** and the converter circuit **210** in order to make the protection circuit **124** fail-proof.

In an implementation, the direction of flow of current from the first stimulation electrode **102A** to the second stimulation electrode **102B** is maintained for a defined time interval based on electroencephalography (EEG) data of the user wearing the wearable headband device **100A.** In another implementation, the direction of flow of current from the second stimulation electrode **102B** to the first stimulation electrode **102A** is maintained for a defined time interval based on the EEG data of the user that is wearing the wearable headband device **100A.** Moreover, the time interval can be different in both situations. In other words, the wearable headband device **100A** may use demographic data, scores on tests and/or EEG data to determine the direction and duration of the defined time interval for electrical current flow between two stimulation electrodes, which are placed on the head. In an example, the defined time interval can vary for different users based on the EEG data of the corresponding user (e.g., two seconds, five seconds, ten seconds, and the like). In another example, the defined time interval can vary for different users based on the FALL-D and other parameters.

In accordance with an embodiment, the plurality of other defined parameters associated with the flow of current from the first electrode **102A** to the second stimulation electrode **102B** and vice-versa comprises amplitude, frequency, phase, ramp-up time, ramp-down time, pulse duration, inter-pulse duration, polarity, and time duration of the flow of current. In an implementation, the plurality of other defined parameters associated with the flow of current form the first electrode to the second simulation electrode **102B** includes the amplitude and the frequency. In another implementation, the plurality of other defined parameters associated with the flow of current from the first electrode to the second stimulation electrode **102B** includes the amplitude, the frequency, the phase, the ramp-up time, and the ramp-down time.

The wearable headband device **100A** is a non-invasive brain stimulation device, which means the wearable headband device **100A** does not require any surgical procedures for non-invasive brain stimulation. The wearable headband device **100A** uses the first circuit **116,** such as the modified H-bridge circuit with low-power switches to switch the direction of current flow between the first stimulation electrode **102A** and the second stimulation electrode **102B,** allowing for a more targeted and effective stimulation with reduced cost. The wearable headband device **100A** can be configured to adapt the current flow direction based on changes in the brain activity patterns, allowing for a more personalized and effective stimulation. Moreover, the wearable headband device **100A** can be customized (e.g., based on the FALL-D parameters) to the individual patient, making it more comfortable, effective, and easy to use. The wearable headband device **100A** further includes the microcontroller **126** for precise current control and the protection circuit **124** to prevent overcurrent flow. The wearable headband device **100A** is designed to be worn as a headband, making it portable and convenient for the user providing greater flexibility and convenience.

FIG. 2 is a diagram that depicts various exemplary components of a brain stimulation circuit, in accordance with an embodiment of the present disclosure. FIG. 2 is described in conjunction with elements from FIG. 1A and 1B. With reference to FIG. 2, there is shown a diagram that includes the first circuit **116** and the protection circuit **124.** There is further shown the first driving circuit **128A** and the second driving circuit **128B** are connected with an AND gate **202,** which is further connected to the microcontroller **126** (e.g., through pins D1 and D2).

In an implementation, the first circuit **116** is a modified H-bridge circuit that includes a first switch **204A** (Q1), a second switch **204B** (Q2), a third switch **204C** (Q3), and a fourth switch **204D** (Q4). Examples of implementation of the first switch **204A,** the second switch **204B,** the third switch **204C,** and the fourth switch **204D** may include but are not limited to low-power transistor switches, such as a p-channel metal-oxide-semiconductor (PMOS) switch, a n-channel metal-oxide-semiconductor (NMOS), and the like. There is further shown that the AND gate **202** is configured to receive a first command from a first pin (e.g., through D1) of the microcontroller **126** to control the first driving circuit **128A.** In addition, the AND gate **202** is further configured to receive a second command from a second pin (e.g., through D2) of the microcontroller **126** to control the second driving circuit **128B.** Moreover, the second driving circuit **128B** is configured to control the second switch **204B,** and the third switch **204C.** In an implementation, a third terminal **216C** of the first switch **204A** is electrically connected to the second switch **204B** through the protection circuit **124,** as shown in FIG. 2. Moreover, the second switch **204B** is electrically connected to the second driving circuit **128B.** Furthermore, the second switch **204B** is electrically connected to the fourth switch **204D** through the second stimulation electrode **102B.** In addition, the third switch **204C** is electrically connected to the fourth switch **204D** through the protection circuit **124,** as shown in FIG. 2.

In an implementation, the third switch **204C** is electrically connected to the second driving circuit **128B,** and the fourth switch **204D** is electrically connected to the first driving circuit **128A.** As a result, the first driving circuit **128A** is configured to control the first switch **204A** of the pair of first type of switches **118** (of FIG. 1B) and further control the fourth switch **204D** of the pair of second type of switches 120 (of FIG. 1B) when the first command is received via the first pin of the microcontroller **126.** In addition, the second driving circuit **128B** is configured to control the second switch **204B** of the pair of first type of switches **118** (of FIG. 1B) and further control the third switch **204C** of the pair of second type of switches **120** (of FIG. 1B) when the second command is received via the second pin of the microcontroller **126.**

In an implementation, the protection circuit **124** includes a Comparator circuit **206,** followed by a converter circuit **210,** and a current bypass circuit **212.** The converter circuit **210** further includes a voltage divider circuit **210A** and a voltage buffer **210B.** Moreover, the Comparator circuit **206** includes an operational amplifier **208** that is connected to the first circuit **116.** In addition, the Comparator circuit **206** of the protection circuit **124** is used to determine an overcurrent passing through the first stimulation electrode **102A** and the second stimulation electrode **102B** based on the potential difference caused across the current sense circuit **130** in protection circuit **124.** Furthermore, in response to an increased potential difference, the protection circuit **124** is configured to cut off the overcurrent passing through the first stimulation electrode **102A** and the second stimulation electrode **102B** independent of receipt of any command from the microcontroller **126.** Moreover, the Comparator Circuit **206** is configured to bypass the current flowing through the first stimulation electrode **102A** and the second stimulation electrode **102B** by providing an alternative path to the current via the current bypass circuit **212** of the protection circuit **124.** In addition, the voltage buffer **210B** is configured to develop an interrupt (INTR) signal for the microcontroller **126** to take further action. In an example, the position of the voltage divider circuit **210A** and the voltage buffer **210B** can be changed without limiting the scope of the present disclosure. Optionally, multiple voltage buffers and multiple voltage divider circuits can be added to improve the efficiency of the protection circuit and have more protection parameters to protect the patients from the extra flow of current.

The voltage divider circuit **210A** is configured to generate INTR signal that is used to prevent the overflow of current when providing stimulation and also ensures the safety of a user by preventing any potential harm from excessive current flow. As a result, the protection circuit **124** is beneficial to ensure the safety of the user by preventing any potential harm, which may be caused by overcurrent passing through the first stimulation electrode **102A** and the second stimulation electrode **102B.** Beneficially as compared to the conventional approach, the Comparator circuit **206** is configured to measure the potential difference across the current sense circuit **130** in the protection circuit **124.** In addition, the protection circuit **124** further provides an interrupt signal to the AND gate **202.** As a result, the AND gate **202** can interrupt the operation of the first stimulation electrode **102A** and the second stimulation electrode **102B** without interruption of microcontroller **126.** The AND gate **202** is an added protection to provide overcurrent-protection in a case where bypass circuit **212** fails to work. The operational amplifier **208** is also connected to a microcontroller pin for microcontroller **126** to continuously monitor voltage as a second-check in overcurrent protection between the first stimulation electrode **102A** and the second stimulation electrode **102B.** The microcontroller **126** further measures voltage and transmit a signal to bypass and shut down the stimulation circuit in case the current overflows.

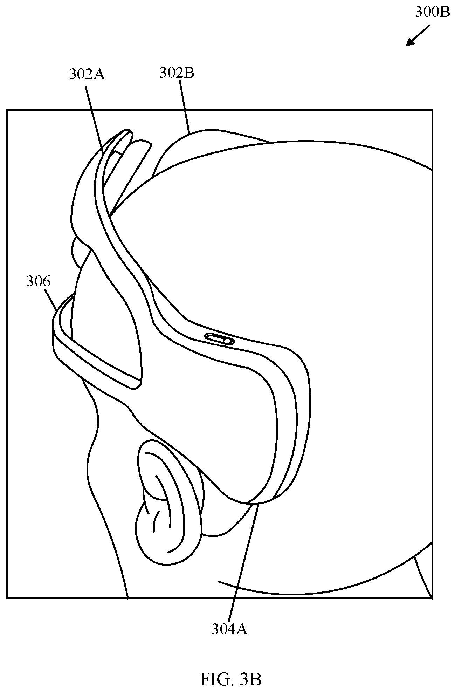

FIGs. 3A and 3B are different diagrams that depict different exemplary scenarios of a wearable headband device for non-invasive brain stimulation to be worn by a user, in accordance with an embodiment of the present disclosure. FIGs. 3A and 3B are described in conjunction with elements from FIGs. 1A, 1B, and 2. With reference to the FIG. 3A, there is shown a diagram **300A** that depicts an exemplary scenario of the wearable headband device **100A** for non-invasive brain stimulation (i.e., a front view). With reference to the FIG. 3B, there is shown a diagram **300B** that depicts an exemplary scenario of the wearable headband device **100A** for non-invasive brain stimulation worn by a user.

The wearable headband device **100A** includes a first-end **302A,** a second-end **302B,** a third-end **304A,** a fourth-end **304B,** and a fifth-end **306.** The first-end **302A** and the third-end **304A** are arranged on the left side of the wearable headband device **100A.** Moreover, the second-end **302B** and the fourth-end **304B** are arranged on the right side of the wearable headband device **100A.** In an example, the first-end **302A** is configured to include the first stimulation electrode **102A,** and the second-end **302B** is configured to include the second stimulation electrode **102B.** In another example, the first-end **302A** is configured to include the second stimulation electrode **102B,** and the second-end **302B** is configured to include the first stimulation electrode **102A.** In addition, the third-end **304A** is configured to enclose or wrap around the second PCB **104B,** and the fourth-end **304B** is configured to enclose the first PCB **104A.** In an example, the third-end **304A** is configured to enclose or wrap around the first PCB **104A,** and the fourth-end **304B** is configured to enclose the second PCB **104B.** In another example, the third-end **304A** is configured to enclose or wrap around the second PCB **104B** as well as to cover the first PCB **104A.** In yet another example, the fourth-end **304B** is configured to cover the first PCB **104A** as well as the second PCB **104B.** Moreover, the fifth-end **306** (e.g., a strap) is configured to cover the connecting part **108** as well as house the EEG circuit **110.** As a result, the fifth-end **306** is used to secure the connecting part **108** and includes the EEG circuit **110** and holds them in place on the patient's head. The wearable headband device **100A** is typically designed to be worn on the head of a user, as shown in FIG. 3B. The wearable headband device **100A** is used for non-invasive brain stimulation, which uses low-intensity electrical currents to stimulate specific regions of the brain and can be used to treat a variety of conditions, including depression, anxiety, MCI, migraine, and the like.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural. The word "exemplary" is used herein to mean "serving as an example, instance or illustration". Any embodiment described as "exemplary" is not necessarily to be construed as preferred or advantageous over other embodiments or to exclude the incorporation of features from other embodiments. The word "optionally" is used herein to mean "is provided in some embodiments and not provided in other embodiments". It is appreciated that certain features of the present disclosure, which are, for clarity, described in the context of separate embodiments, may also be provided in combination in a single embodiment. Conversely, various features of the invention, which are, for brevity, described in the context of a single embodiment, may also be provided separately or in any suitable combination or as suitable in any other described embodiment of the disclosure.

## Claims

1. A wearable headband device **(100A)** for non-invasive brain stimulation, comprising:
a first stimulation electrode **(102A)** and a second stimulation electrode **(102B);** and
an electronic circuit **(106)** for switching a direction of flow of current between the first stimulation electrode **(102A)** and the second stimulation electrode **(102B),** wherein the electronic circuit **(106)** comprises:
a first circuit **(116)** that comprises a pair of first type of switches **(118)** and a pair of second type of switches **(120)** that are electrically connected to each other, wherein the first circuit **(116)** is further electrically connected to the first stimulation electrode **(102A)** and the second stimulation electrode **(102B);**
a plurality of second circuits **(122)** configured to control an operation of the pair of first type of switches **(118)** and the pair of second type of switches **(120)** of the first circuit **(116);**
a protection circuit **(124)** that is configured to detect and control the flow of current passing through the first stimulation electrode **(102A)** and the second stimulation electrode **(102B)** via the first circuit **(116),** based on a voltage drop across a current sense circuit **(130)** of the protection circuit **(124);** and
a microcontroller **(126)** that is configured to control the pair of first type of switches **(118)** and the pair of second type of switches **(120)** of the first circuit **(116)** through the plurality of second circuits **(122)** for the switching the direction of flow of current between the first stimulation electrode **(102A)** and the second stimulation electrode **(102B)** in the non-invasive brain stimulation.

2. The wearable headband device **(100A)** of claim 1, wherein the plurality of second circuits **(122)** comprises a first driving circuit **(128A)** that is configured to control a first switch **(204A)** of the pair of first type of switches **(118)** and further control a fourth switch **(204D)** of the pair of second type of switches **(120)** when a first command is received via a first pin of the microcontroller **(126).**

3. The wearable headband device **(100A)** of claim 1, wherein the plurality of second circuits **(122)** comprises a second driving circuit **(128B)** configured to control a second switch **(204B)** of the pair of first type of switches **(118)** and further control a fourth switch **(204C)** of the pair of second type of switches **(120)** when a second command is received via a second pin of the microcontroller **(126).**

4. The wearable headband device **(100A)** of claim 1, wherein the first circuit **(116)** is a modified H-bridge circuit.

5. The wearable headband device **(100A)** of claim 1, wherein in the first circuit **(116),** a first terminal **(216A)** of the first switch **(204A)** is electrically connected to a third terminal **(216C)** of the third switch **(204C)** through the first stimulation electrode **(102A),** a second terminal **(216B)** of the first switch **(204A)** is electrically connected to the first driving circuit **(128A),** and a third terminal **(216C)** of the first switch **(204A)** is electrically connected to a third terminal **(216C)** of the second switch **(204B)** through the protection circuit **(124),** wherein in the first circuit **(116),** a first terminal **(216A)** of the second switch **(204B)** is electrically connected to a third terminal **(216C)** of the fourth switch **(204D)** through the second stimulation electrode **(102B),** and the second terminal **(216B)** of the second switch **(204B)** is electrically connected to the second driving circuit **(128B),** wherein in the first circuit **(116),** a first terminal **(216A)** of the third switch **(204C)** is electrically connected to a first terminal **(216A)** of the fourth switch **(204D)** through the protection circuit **(124),** a second terminal **(216B)** of the third switch **(204C)** is electrically connected to the second driving circuit **(128B),** and a second terminal **(216B)** of the fourth switch **(204D)** is electrically connected to the first driving circuit **(128A).**

6. The wearable headband device **(100A)** of claim 1, the first circuit **(116)** further connected with a battery **(112)** that acts as a power source, and wherein the first circuit **(116)** is configured to steer the flow of current from the power source toward the first stimulation electrode **(102A)** and the second stimulation electrode **(102B)** via the pair of first type of switches **(118)** and the pair of second type of switches **(120)** when the wearable headband device **(100A)** receives a command from a user through the microcontroller **(126).**

7. The wearable headband device **(100A)** of claim 6, wherein the flow of current that passes through the first stimulation electrode **(102A)** and the second stimulation electrode **(102B)** causes a potential difference across the current sense circuit **(130)** of the protection circuit **(124),** wherein the current sense circuit **(130)** that is configured to determine an overcurrent passing through the first stimulation electrode **(102A)** and the second stimulation electrode **(102B)** based on the potential difference caused across the current sense circuit **(130)** of the protection circuit **(124).**

8. The wearable headband device **(100A)** of claim 7, wherein the protection circuit **(124)** is further configured to determine if a voltage drop across the current sense circuit **(130)** of protection circuit **(124)** exceeds a pre-defined threshold, and in response to an increased voltage drop, the protection circuit **(124)** is configured to cut off the overcurrent passing through the first stimulation electrode **(102A)** and the second stimulation electrode **(102B),** wherein if the voltage drop across the current sense circuit **(130)** of protection circuit **(124)** exceeds the pre-defined threshold, then the protection circuit **(124)** is configured to cut off the overcurrent passing to the first stimulation electrode **(102A)** and the second stimulation electrode **(102B)** independent of receipt of any command from the microcontroller **(126),** wherein the protection circuit **(124)** is configured to cut off the overcurrent passing to the first stimulation electrode **(102A)** and the second stimulation electrode **(102B)** without depending on a polarity of the flow of current across the first stimulation electrode **(102A)** and the second stimulation electrode **(102B)** .

9. The wearable headband device **(100A)** of claim 8, wherein the protection circuit **(124)** comprises a Comparator circuit **(206)** and a converter circuit (210) that further includes a voltage divider circuit **(210A),** a voltage buffer **(210B).**

10. The wearable headband device **(100A)** of claim 1, wherein the protection circuit **(124)** is further configured to cut off the overcurrent passing through the first stimulation electrode **(102A)** and the second stimulation electrode **(102B),** and the comparator circuit **(206)** is configured to bypass the current flowing through the first stimulation electrode **(102A)** and the second stimulation electrode **(102B)** by providing an alternative path to the current via current bypass circuit **(212)** of the protection circuit **(124).**

11. The wearable headband device **(100A)** of claim 1, wherein the protection circuit **(124)** is further configured to provide overvoltage protection between the first stimulation electrode **(102A)** and the second stimulation electrode **(102B).**

12. The wearable headband device **(100A)** of claim 1, wherein the switching of the direction of flow of current further comprises reversing a polarity or the direction of the flow of current between the first stimulation electrode **(102A)** and the second stimulation electrode **(102B)** or changing at least one parameter of a plurality of other defined parameters associated with the flow of current.

13. The wearable headband device **(100A)** of claim 11, wherein the direction and the plurality of other defined parameters associated with the flow of current from the first stimulation electrode **(102A)** to the second stimulation electrode **(102B)** is maintained for a defined time interval based on demographic data, scores on tests and electroencephalography (EEG) data of a user wearing the wearable headband device **(100A),** wherein the plurality of other defined parameters associated with the flow of current from the first electrode to the second stimulation electrode **(102B)** comprises amplitude, frequency, phase, ramp-up time, ramp-down time, pulse duration, inter-pulse duration, polarity, and time duration of the flow of current.

14. The wearable headband device **(100A)** of claim 1, wherein the microcontroller **(126)** is further configured to detect a fault in the electronic circuit **(106)** and as a second check on the protection circuit **(124)** and in response open the pair of first type of switches **(118)** and the pair of second type of switches **(120)** of the first circuit **(116)** in response to the fault.

15. The wearable headband device **(100A)** of claim 1, wherein the switching an amount of the current flow is performed based on demographic data, scores on tests or a change in a pattern of electroencephalography (EEG) data and based on one or more transcranial electrical stimulation (tES) application criteria when wearable headband device **(100A)** is worn by a user.
